# EUROPEAN PATENT APPLICATION

(11) **EP 1 167 329 A2**
(43) Date of publication of application: **02.01.2002**
(21) Application number: 01305272.5
(22) Date of filing: 18.06.2001
(51) Int. Cl.: C07C 29/04, C07F 9/50, C07C 29/12, C07D 211/42, C07C 35/08, C07C 35/28, C07C 35/36, C07C 33/46, C07C 33/20

(54) **Oxidation of carbon-boron bonds**

(30) Priority: 26.06.2000 US 214109 P
(71) Applicant: Pfizer Products Inc., Groton, Connecticut 06340 (US)
(72) Inventor: Cai, Weiling, Pfizer Global, Groton, Connecticut 06340 (US); Ripin, David Harold Brown, Pfizer Global, Groton, Connecticut 06340 (US)
(74) Representative: Ruddock, Keith Stephen

(57) **Abstract**

The present invention relates to a scaleable process for the oxidation of carbon-boron bonds with Oxone®.

## Description

The present invention relates to a scaleable process for the oxidation of carbon-boron bonds with Oxone® .

The oxidation of carbon-boron bonds to the corresponding alcohol can be achieved with a wide range of oxidants. The most common method used is hydrogen peroxide in a basic medium, although trimethylamine N-oxide (TMANO), peracids, chromium reagents, sodium perborate, sodium percarbonate, and many others can be used. Most of these methods are of limited utility on large scale due to safety, reproducibility, or reagent waste concerns.

The use of Oxone® in the oxidation of carbon-boron bonds provides higher yields and purity than any of the other procedures investigated. Oxone®, in direct comparison, has a higher onset of decomposition than 30% hydrogen peroxide and liberates less energy. This reaction is performed at lower temperature, which provides a larger margin of safety, and is dose-rate controlled by addition of Oxone® solution, providing a better overall safety profile for the oxidation. Additionally, Oxone® is a solid, allowing for the addition of precisely weighed amounts of reagent to be used in the reaction.

### Summary of the Invention

The present invention relates to a process for preparing a compound of the formula 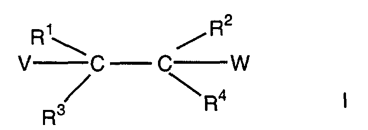 wherein V and W are each independently selected from hydrogen or hydroxy;
R¹, R², R³ and R⁴ are each independently selected from the group consisting of hydrogen, deuterium, amino, halo, hydoxy, nitro, carboxy, trifluoromethyl, trifluoromethoxy, (C₁-C₆)alkyl, (C₁-C₆)alkoxy, (C₃-C₁₀)cycloalkyl wherein the alkyl, alkoxy or cycloalkyl groups are optionally substituted by one to three groups selected from halo, hydroxy, carboxy, amino, (C₁-C₆)alkylamino, ((C₁-C₆)alkyl)₂amino, (C₅-C₉)heteroaryl, (C₂-C₉)heterocycloalkyl, (C₃-C₉)cycloalkyl or (C₆-C₁₀)aryl; or R¹, R², R³ and R⁴ are each independently (C₃-C₁₀)cycloalkyl, (C₃-C₁₀)cycloalkoxy, (C₁-C₆)alkylamino, ((C₁-C₆)alkyl)₂amino, (C₆-C₁₀)arylamino, (C₁-C₆)alkylsulfinyl, (C₆-C₁₀)arylsulfinyl, (C₁-C₆)alkylsulfonyl, (C₆-C₁₀)arylsulfonyl, (C₁-C₆)acyl, (C₁-C₆)alkoxy-CO-NH-, (C₁-C₆)alkyamino-CO-, (C₅-C₉)heteroaryl, (C₂-C₉)heterocycloalkyl or (C₆-C₁₀)aryl wherein the heteroaryl, heterocycloalkyl and aryl groups are optionally substituted by one to three halo, (C₁-C₆)alkyl, (C₁-C₆)alkyl-CO-NH-, (C₁-C₆)alkoxy-CO-NH-, (C₁-C₆)alkyl-CO-NH-(C₁-C₆)alkyl, (C₁-C₆)alkoxy-CO-NH-(C₁-C₆)alkyl, (C₁-C₆)alkoxy-CO-NH-(C₁-C₆)alkoxy, carboxy, carboxy(C₁-C₆)alkyl, carboxy(C₁-C₆)alkoxy, benzyloxycarbonyl(C₁-C₆)alkoxy, (C₁-C₆)alkoxycarbonyl(C₁-C₆)alkoxy, (C₆-C₁₀)aryl, amino, amino(C₁-C₆)alkyl, (C₁-C₆)alkoxycarbonylamino, (C₆-C₁₀)aryl(C₁-C₆)alkoxycarbonylamino, (C₁-C₆)alkylamino, ((C₁-C₆)alkyl)₂amino, (C₁-C₆)alkylamino(C₁-C₆)alkyl, ((C₁-C₆)alkyl)₂amino(C₁-C₆)alkyl, hydroxy, (C₁-C₆)alkoxy, carboxy, carboxy(C₁-C₆)alkyl, (C₁-C₆)alkoxycarbonyl, (C₁-C₆)alkoxycarbonyl(C₁-C₆)alkyl, (C₁-C₆)alkoxy-CO-NH-, (C₁-C₆)alkyl-CO-NH-, cyano, (C₅-C₉)heterocycloalkyl, amino-CO-NH-, (C₁-C₆)alkylamino-CO-NH-, ((C₁-C₆)alkyl)₂amino-CO-NH-, (C₆-C₁₀)arylamino-CO-NH-, (C₅-C₉)heteroarylamino-CO-NH-, (C₁-C₆)alkylamino-CO-NH-(C₁-C₆)alkyl, ((C₁-C₆)alkyl)₂amino-CO-NH-(C₁-C₆)alkyl, (C₆-C₁₀)arylamino-CO-NH-(C₁-C₆)alkyl, (C₅-C₉)heteroarylamino-CO-NH-(C₁-C₆)alkyl, (C₁-C₆)alkylsulfonyl, (C₁-C₆)alkylsulfonylamino, (C₁-C₆)alkylsulfonylamino(C₁-C₆)alkyl, (C₆-C₁₀)arylsulfonyl, (C₆-C₁₀)arylsulfonylamino, (C₆-C₁₀)arylsulfonylamino(C₁-C₆)alkyl, (C₁-C₆)alkylsulfonylamino, (C₁-C₆)alkylsulfonylamino(C₁-C₆)alkyl, (C₅-C₉)heteroaryl or (C₂-C₉)heterocycloalkyl;
or R¹ and R², and/or R³ and R⁴, and/or R¹ and R³, and/or R² and R⁴ are taken together with the carbon to which they are attached to form a (C₃-C₁₀)cycloalkyl or (C₂-C₉)heterocycloalkyl group optionally substituted by one to five groups consisting of carboxy, cyano, amino, deuterium, hydroxy, (C₁-C₆)alkyl, (C₁-C₆)alkoxy, halo, (C₁-C₆)acyl, (C₁-C₆)alkylamino, amino(C₁-C₆)alkyl, (C₁-C₆)alkoxy-CO-NH, (C₁-C₆)alkylamino-CO-, (C₂-C₆)alkenyl, (C₂-C₆) alkynyl, (C₁-C₆)alkylamino, amino(C₁-C₆)alkyl, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R¹⁵R¹⁶N-CO-O-, R¹⁵R¹⁶N-CO-(C₁-C₆)alkyl, (C₁-C₆)alkyl-S(O)ₘ, R¹⁵R¹⁶NS(O)ₘ, R¹⁵R¹⁶NS(O)ₘ (C₁-C₆)alkyl, R¹⁵S(O)ₘ R¹⁶N, R¹⁵S(O)ₘR¹⁶N(C₁-C₆)alkyl wherein m is 1 or 2 and R¹⁵ and R¹⁶ are each independently selected from hydrogen or (C₁-C₆)alkyl; and a group of the formula 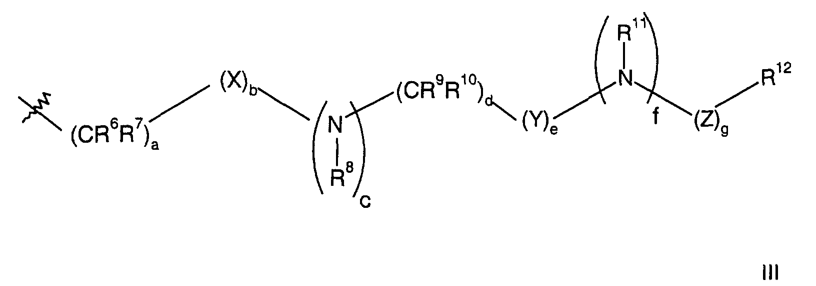 wherein a is 0, 1, 2, 3 or 4;
   b, c, e, f and g are each independently 0 or 1;
   d is 0,1, 2, or 3;
   X is S(O)ₙ wherein n is 1 or 2; oxygen, carbonyl or -C(=N-cyano)-;
   Y is S(O)ₙ wherein n is 1 or 2; or carbonyl; and
   Z is carbonyl, C(O)O-, C(O)NR- or S(O)ₙ wherein n is 1 or 2;
   R⁶, R⁷, R⁸, R⁹, R¹⁰ and R¹¹ are each independently selected from the group consisting of hydrogen or (C₁-C₆)alkyl optionally substituted by deuterium, hydroxy, amino, trifluoromethyl, (C₁-C₆)acyloxy, (C₁-C₆)acylamino, (C₁-C₆)alkylamino, ((C₁-C₆)alkyl)₂amino, cyano, cyano(C₁-C₆)alkyl, trifluoromethyl(C₁-C₆)alkyl, nitro, nitro(C₁-C₆)alkyl or (C₁-C₆)acylamino;
   R¹² is carboxy, cyano, amino, oxo, deuterium, hydroxy, trifluoromethyl, (C₁-C₆)alkyl, trifluoromethyl(C₁-C₆)alkyl, (C₁-C₆)alkoxy, halo, (C₁-C₆)acyl, (C₁-C₆)alkylamino, ((C₁-C₆)alkyl)₂ amino, amino(C₁-C₆)alkyl, (C₁-C₆)alkoxy-CO-NH, (C₁-C₆)alkylamino-CO-, (C₁-C₆)alkylamino, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R¹⁵R¹⁶N-CO-O-, R¹⁵R¹⁶N-CO-(C₁-C₆)alkyl, R¹⁵C(O)NH, R¹⁵OC(O)NH, R¹⁵NHC(O)NH, (C₁-C₆)alkyl-S(O)ₘ, (C₁-C₆)alkyl-S(O)ₘ-(C₁-C₆)alkyl, R¹⁵R¹⁶NS(O)ₘ, R¹⁵R¹⁶NS(O)ₘ (C₁-C₆)alkyl, R¹⁵S(O)ₘ R¹⁶N, R¹⁵S(O)ₘR¹⁶N(C₁-C₆)alkyl wherein m is 1 or 2 and R¹⁵ and R¹⁶ are each independently selected from hydrogen or (C₁-C₆)alkyl; or R¹² is (C₆-C₁₀)aryl, (C₂-C₉)heteroaryl, (C₃-C₁₀)cycloalkyl, (C₂-C₉)heterocycloalkyl, wherein the aryl, heteroaryl, cycloalkyl and heterocycloalkyl groups are optionally substituted by one to four groups consisting of hydrogen, deuterium, halo, (C₁-C₆)alkyl, (C₁-C₆)alkyl-CO-NH-, (C₁-C₆)alkoxy-CO-NH-, (C₁-C₆)alkyl-CO-NH-(C₁-C₆)alkyl, (C₁-C₆)alkoxy-CO-NH-(C₁-C₆)alkyl, (C₁-C₆)alkoxy-CO-NH-(C₁-C₆)alkoxy, carboxy, carboxy(C₁-C₆)alkyl, carboxy(C₁-C₆)alkoxy, benzyloxycarbonyl(C₁-C₆)alkoxy, (C₁-C₆)alkoxycarbonyl(C₁-C₆)alkoxy, (C₆-C₁₀)aryl, amino, amino(C₁-C₆)alkyl, (C₁-C₆)alkoxycarbonylamino, (C₆-C₁₀)aryl(C₁-C₆)alkoxycarbonylamino, (C₁-C₆)alkylamino, ((C₁-C₆)alkyl)₂amino, (C₁-C₆)alkylamino(C₁-C₆)alkyl, ((C₁-C₆)alkyl)₂amino(C₁-C₆)alkyl, hydroxy, (C₁-C₆)alkoxy, carboxy, carboxy(C₁-C₆)alkyl, (C₁-C₆)alkoxycarbonyl, (C₁-C₆)alkoxycarbonyl(C₁-C₆)alkyl, (C₁-C₆)alkoxy-CO-NH-, (C₁-C₆)alkyl-CO-NH-, cyano, (C₅-C₉)heterocycloalkyl, amino-CO-NH-, (C₁-C₆)alkylamino-CO-NH-, ((C₁-C₆)alkyl)₂amino-CO-NH-, (C₆-C₁₀)arylamino-CO-NH-, (C₅-C₉)heteroarylamino-CO-NH-, (C₁-C₆)alkylamino-CO-NH-(C₁-C₆)alkyl, ((C₁-C₆)alkyl)₂amino-CO-NH-(C₁-C₆)alkyl, (C₆-C₁₀)arylamino-CO-NH-(C₁-C₆)alkyl, (C₅-C₉)heteroarylamino-CO-NH-(C₁-C₆)alkyl, (C₁-C₆)alkylsulfonyl, (C₁-C₆)alkylsulfonylamino, (C₁-C₆)alkylsulfonylamino(C₁-C₆)alkyl, (C₆-C₁₀)arylsulfonyl, (C₆-C₁₀)arylsulfonylamino, (C₆-C₁₀)arylsulfonylamino(C₁-C₆)alkyl, (C₁-C₆)alkylsulfonylamino, (C₁-C₆)alkylsulfonylamino(C₁-C₆)alkyl, (C₅-C₉)heteroaryl or (C₂-C₉)heterocycloalkyl;
   with the proviso that X and Y cannot both be hydrogen or hydroxy;
      comprising reacting a compound of the formula 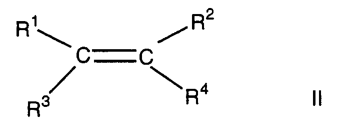 wherein R¹, R², R³ and R⁴ are as defined above, with a borane, followed by reacting the intermediate so formed with Oxone®.

The term "Oxone®" is a name of a monopersulfate compound used in this invention, having the formula 2KHSO₅·KHSO₄·K₂SO₄, and sold by Aldrich Chemical Company, P.O. Box 2060, Milwaukee, WI 53201, USA.

The term "alkyl", as used herein, unless otherwise indicated, includes saturated monovalent hydrocarbon radicals having straight or branched moieties or combinations thereof.

The term "alkoxy", as used herein, includes O-alkyl groups wherein "alkyl" is defined above.

The term "halo", as used herein, unless otherwise indicated, includes fluoro, chloro, bromo or iodo.

Unless otherwise indicated, the alkyl groups referred to herein, as well as the alkyl moieties of other groups referred to herein (e.g., alkoxy), may be linear or branched, and they may also be cyclic (e.g., cyclopropyl, cyclobutyl, cyclopentyl, cyclohexyl or cycloheptyl) or be linear or branched and contain cyclic moieties. Unless otherwise indicated, halogen includes fluorine, chlorine, bromine, and iodine.

(C₂-C₉)Heterocycloalkyl when used herein refers to pyrrolidinyl, tetrahydrofuranyl, dihydrofuranyl, tetrahydropyranyl, pyranyl, thiopyranyl, aziridinyl, oxiranyl, methylenedioxyl, chromenyl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, chromanyl, etc. One of ordinary skill in the art will understand that the connection of said (C₂-C₉)heterocycloalkyl rings is through a carbon or a sp³ hybridized nitrogen heteroatom.

(C₂-C₉)Heteroaryl when used herein refers to furyl, thienyl, thiazolyl, pyrazolyl, isothiazolyl, oxazolyl, isoxazolyl, pyrrolyl, triazolyl, tetrazolyl, imidazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridyl, pyrimidyl, pyrazinyl, pyridazinyl, 1,2,4-triazinyl, 1,2,3-triazinyl, 1,3,5-triazinyl, pyrazolo[3,4-b]pyridinyl, cinnolinyl, pteridinyl, purinyl, 6,7-dihydro-5H-[1]pyrindinyl, benzo[b]thiophenyl, 5, 6, 7, 8-tetrahydro-quinolin-3-yl, benzoxazolyl, benzothiazolyl, benzisothiazolyl, benzisoxazolyl, benzimidazolyl, thianaphthenyl, isothianaphthenyl, benzofuranyl, isobenzofuranyl, isoindolyl, indolyl, indolizinyl, indazolyl, isoquinolyl, quinolyl, phthalazinyl, quinoxalinyl, quinazolinyl, benzoxazinyl; etc. One of ordinary skill in the art will understand that the connection of said (C₂-C₉)heterocycloalkyl rings is through a carbon atom or a sp³ hybridized nitrogen heteroatom.

(C₆-C₁₀)aryl when used herein refers to phenyl or naphthyl.
ediate so formed with Oxone®.
The present invention relates to a process wherein the borane is borane,
diborane, or (C₁-C₁₂)alkylborane.
The present invention further relates to a process wherein R¹, R², R³ and R⁴ are each independently selected from hydrogen, deuterium, (C₃-C₁₀)cycloalkyl, (C₃-C₁₀)cycloalkoxy, (C₁-C₆)alkylsulfonyl, (C₆-C₁₀)arylsulfonyl, (C₁-C₆)acyl, (C₃-C₁₀)cycloalkyl, (C₅-C₉)heteroaryl, (C₂-C₉)heterocycloalkyl, (C₆-C₁₀)aryl, wherein heteroaryl, heterocycloalkyl, aryl groups are optionally substituted by one to three halo, (C₁-C₆)alkyl or (C₁-C₆)alkoxy.

The present invention further relates to a process, wherein R¹ and R², or R³ and R⁴, R¹ and R³, or R² and R⁴ are taken together with the carbon to which they are attached to form a (C₃-C₁₀)cycloalkyl or (C₂-C₉)heterocycloalkyl group optionally substituted by one to five groups consisting of carboxy, cyano, amino, deuterium, hydroxy, (C₁-C₆)alkyl, (C₁-C₆)alkoxy, halo, (C₁-C₆)acyl, (C₁-C₆)alkylamino, amino(C₁-C₆)alkyl, (C₁-C₆)alkoxy-CO-NH, (C₁-C₆)alkylamino-CO-, (C₂-C₆)alkenyl, (C₂-C₆) alkynyl, (C₁-C₆)alkylamino, amino(C₁-C₆)alkyl, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R¹⁵R¹⁶N-CO-O-, R¹⁵R¹⁶N-CO-(C₁-C₆)alkyl, (C₁-C₆)alkyl-S(O)ₘ, R¹⁵R¹⁶NS(O)ₘ, R¹⁵R¹⁶NS(O)ₘ (C₁-C₆)alkyl, R¹⁵S(O)ₘ R¹⁶N, R¹⁵S(O)ₘR¹⁶N(C₁-C₆)alkyl wherein m is 1 or 2 and R¹⁵ and R¹⁶ are each independently selected from hydrogen or (C₁-C₆)alkyl; and a group of the formula 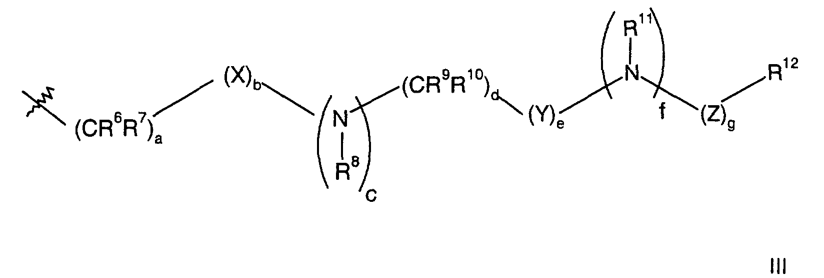 wherein a is 0, 1, 2, 3 or 4;
b, c, e, f and g are each independently 0 or 1;
d is 0, 1, 2, or 3;
X is S(O)ₙ wherein n is 1 or 2; oxygen, carbonyl or -C(=N-cyano)-;
Y is S(O)ₙ wherein n is 1 or 2; or carbonyl; and
Z is carbonyl, C(O)O-, C(O)NR- or S(O)ₙ wherein n is 1 or 2;
R⁶, R⁷, R⁸, R⁹, R¹⁰ and R¹¹ are each independently selected from the group consisting of hydrogen or (C₁-C₆)alkyl optionally substituted by deuterium, hydroxy, amino, trifluoromethyl, (C₁-C₆)acyloxy, (C₁-C₆)acylamino, (C₁-C₆)alkylamino, ((C₁-C₆)alkyl)₂amino, cyano, cyano(C₁-C₆)alkyl, trifluoromethyl(C₁-C₆)alkyl, nitro, nitro(C₁-C₆)alkyl or (C₁-C₆)acylamino.

The present invention further relates to a process wherein a is 0; b is 1; X is carbonyl; c is 0; d is 0; e is 0; f is 0; and g is 0.

The present invention further relates to a process wherein a is 0; b is 1; X is carbonyl; c is 0; d is 1; e is 0; f is 0, and g is 0.

The present invention further relates to a process wherein a is 0; b is 1; X is carbonyl; c is 1; d is 0; e is 0; f is 0; and g is 0.

The present invention further relates to a process wherein a is 0; b is 1; X is-C(=N=cyano)-; c is 1; d is 0; e is 0; f is 0; and g is 0.

The present invention further relates to a process wherein a is 0; b is 0; c is 0; d is 0; e is 0; f is 0; g is 1; and Z is -C(O)-O-.

The present invention further relates to a process wherein a is 0; b is 1; X is S(O)ₙ; n is 2; c is 0; d is 0; e is 0; f is 0; and g is 0.

The present invention further relates to a process wherein a is 0; b is 1; X is S(O)ₙ; n is 2; c is 0; d is 2; e is 0; f is 1; g is 1; and Z is carbonyl.

The present invention further relates to a process wherein a is 0; b is 1; X is S(O)ₙ; n is 2; c is 0; d is 2; e is 0; f is 1; and g is 0.

The present invention further relates to a process wherein a is 0; b is 1; X is carbonyl; c is 1; d is 0; e is 1; Y is S(O)ₙ; n is 2; f is 0; and g is 0.

The present invention further relates to a process wherein a is 0; b is 1; X is S(O)ₙ; n is 2; c is 1; d is 0; e is 0; f is 0; and g is 0.

The present invention further relates to a process wherein a is 1; b is 1; X is carbonyl; c is 1; d is 0; e is 0; f is 0; and g is 0.

The present invention further relates to a process wherein a is 0; b is 1; X is S(O)ₙ; c is 0; d is 1; e is 1; Y is S(O)ₙ; n is 2; f is 0; and g is 0.

The present invention further relates to a process wherein a is 0; b is 1; X is S(O)ₙ; c is 0; d is 2, 3 or 4; e is 1; Y is S(O)ₙ; n is 2; f is 1; and g is 0.

The present invention further relates to a process wherein a is 0; b is 1; X is oxygen; c is 0; d is 2, 3 or 4; e is 1; Y is S(O)ₙ; n is 2; f is 1; and g is 0.

The present invention further relates to a process wherein a is 0; b is 1; X is oxygen; c is 0; d is 2, 3 or 4; e is 1; Y is S(O)ₙ; n is 2; f is 0; and g is 0.

The present invention further relates to a process wherein a is 0; b is 1; X is carbonyl; c is 1; d is 2, 3 or 4; e is 1; Y is S(O)ₙ; f is 0; and g is 0.

The present invention further relates to a process wherein a is 0; b is 1; X is carbonyl; c is 1; d is 2, 3 or 4; e is 1; Y is S(O)ₙ; n is 2; f is 1; and g is 0.

The present invention further relates to a process wherein R¹² is cyano, deuterium, hydroxy, trifluoromethyl, (C₁-C₆)alkyl, triflouromethyl(C₁-C₆)alkyl, (C₁-C₆)alkoxy, halo, (C₁-C₆)acyl, (C₆-C₁₀)aryl, (C₂-C₉)heteroaryl, (C₃-C₁₀)cycloalkyl, or (C₂-C₉)heterocycloalkyl.

### Detailed Description of the Invention

The following reaction Scheme illustrates the preparation of the compounds of the present invention. Unless otherwise indicated V, W, R¹, R², R³ and R⁴ in the reaction Scheme and the discussion that follow are defined as above.

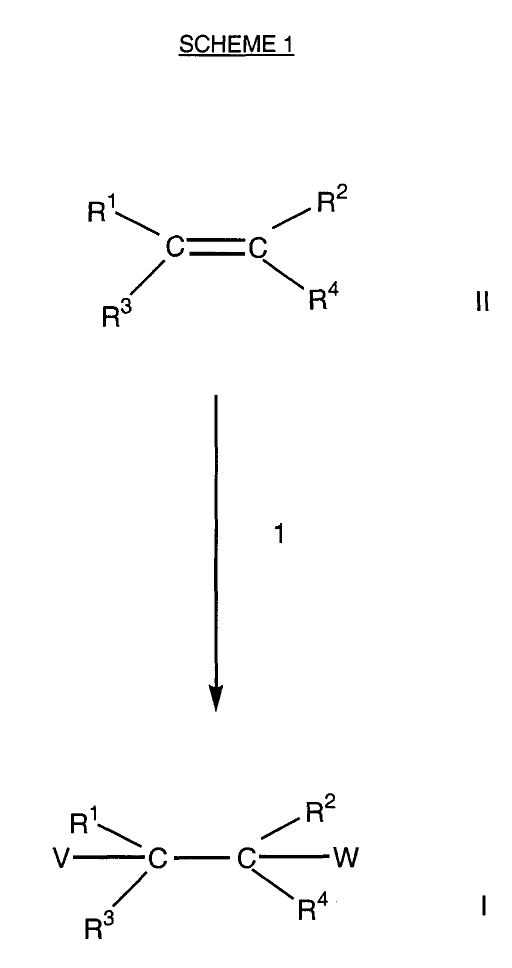

In reaction 1 of Scheme 1, the compound of formula **II** is converted to the corresponding compound of formula **I** by first reacting **II** with a borane, such as borane, diborane or an alkylborane, in the presence of a polar aprotic solvent, such as tetrahydrofuran, dioxane, diethylether, dichloromethane or dichloroethane, preferably tetrahydrofuran. The carbon-boron bond of the intermediate so formed is then oxidized by treating the intermediate with Oxone® . The Oxone® oxidation is usually carried out using the same polar aprotic solvent used in the hydroboration reaction, however, in those cases where a different solvent is used a phase transfer catalyst, such as tetrabutyl ammonium bromide, is added to speed the reaction. Preferably, the entire reaction is done in tetrahydrofuran and no phase transfer catalyst is needed. The Oxone® oxidation is carried out at a temperature between about -15°C to about 100°C, preferably about 0°C to about 35°C, for a time period between about 5 minutes to about 72 hours, preferably about 8 to 12 hours.

The following example illustrates the preparation of a compound of the present invention but it is not limited to the details thereof. NMR data are reported in parts per million (δ) and are referenced to the deuterium lock signal from the sample solvent (deuteriochloroform unless otherwise specified). Commercial reagents were utilized without further purification. Room or ambient temperature refers to 20-25°C.

### Example 1

### trans-1-Hydroxy-2-methylcyclohexane

To a solution of 1-methylcyclohexene (1.03 grams, 10.7 mmol) in tetrahydrofuran (15 mL) was added sodium borohydride (0.64 grams, 17.0 mmol) at room temperature under nitrogen. The slurry was cooled to 0°C, and BF₃·OEt₂ (1.5 mL, 1.70 grams, 12.0 mmol) in tetrahydrofuran (2.5 mL) was slowly added through an addition funnel. The addition was kept slow enough to keep the temperature of the reaction mixture below 0°C. After the addition; the reaction mixture was stirred at 0°C for 1 hour and room temperature for 1.5 hours. The reaction was re-cooled to 0°C and water (10.0 mL) was added slowly to destroy the excess borane. The reaction was stirred at room temperature for 2 hours, followed by the addition of Oxone® (21.2 grams, 34.8 mmol) in water (40 mL) at 0°C. The reaction mixture was allowed to warm to room temperature and stirred overnight. The reaction was quenched by adding NaHSO₃ (solid) until all excess oxidant was destroyed (potassium iodine/starch test paper). The pH of the reaction mixture was 1-2 and was adjusted to pH of 7 with 2N sodium hydroxide and extracted with ethyl acetate (4 times with 25 mL). The organic layer was washed with brine, dried over NaSO₄, and concentrated *in vacuo.* The residue obtained was subjected for a flash chromatography column eluting with ethyl alcohol/hexane (1:5) to give the pure alcohol *trans*-1-hydroxy-2-methylcyclohexane (0.62 grams, 50%) NMR: 3.1(m,1H), 2.1(s,br,1H), 1.9(m,1H), 1.6(m, 4H), 1.2(m, 4H), 0.9(d,3H).

### Example 2

### ((1α,2β,3α,5α)]-2,6,6-Trimethyl-Bicyclo[3.1.1]heptan-3-ol

To a solution of 2,6,6-trimethylbicyclo[3.1.1]hept-2-ene (1.36 grams, 10.0 mmol) in tetrahydrofuran (15 mL) was added sodium borohydride (0.64 grams, 17.0 mmol) at room temperature under nitrogen. The slurry was cooled to 0°C, and BF₃·OEt₂ (1.5 mL, 1.70 grams, 12.0 mmol) in tetrahydrofuran (2.5 mL) was slowly added through an addition funnel. The addition was kept slow enough to keep the temperature of the reaction mixture below 0°C. After the addition; the reaction mixture was stirred at 0°C for 1 hour and room temperature for 1.5 hour. The reaction was re-cooled to 0°C and water (10.0 mL) was added slowly to destroy the excess borane. The reaction was stirred at room temperature for 2 hours, followed by the addition of Oxone® (21.6 grams, 34.8 mmol) in water (40 mL) at 0°C. The reaction mixture was allowed to warm to room temperature and stirred overnight. The reaction was quenched by adding NaHSO₃ (solid) until all excess oxidant was destroyed (Kl/starch test paper). The pH of the reaction mixture was 1-2. and was neutralized with 2N sodium hydroxide and extracted with ethyl acetate (4 times with 25 mL). The organic layer was washed with brine, dried over NaSO₄, and concentrated *in vacuo*. The residue obtained was the pure alcohol ((1α,2β,3α,5α)]-2,6,6-trimethyl-Bicyclo[3.1.1]heptan-3-ol (1.50 grams, 95%).NMR: 4.1(m,3H), 2.5(m,1H), 2.3(m,1H), 2.0(s,br,1H), 1.9(m,1H), 1.8(m, 1H), 1.7(m,1H), 1.2(s,3H), 1,1(d,3H), 1.0(d,1H), 0.9(s, 3H).

### Example 3

### cis-Bicyclo[4.4.0]decan-1-ol

To a solution of 9,10-Dehydrodecalin (1.46 grams, 10.7 mmol) in tetrahydrofuran (15 mL) was added sodium borohydride (0.70 grams, 18.5 mmol) at room temperature under nitrogen. The slurry was cooled to 0°C, and BF₃·OEt₂ (1.8 mL, 2.01 grams, 14.2 mmol) in tetrahydrofuran (3.0 mL) was slowly added through an addition funnel. The addition was kept slow enough to keep the temperature of the reaction mixture below 0°C. After the addition; the reaction mixture was stirred at 0°C for 1 hour and room temperature for 1.5 hours. The reaction was re-cooled to 0°C and water (10.0 mL) was added slowly to destroy the excess borane. The reaction was stirred at room temperature for 2 hours, followed by the addition of Oxone® (21.6 grams, 34.8 mmol) in water (40 mL) at 0°C. The reaction mixture was allowed to warm to room temperature and stirred overnight. The reaction was quenched by adding NaHSO₃ (solid) until all excess oxidant was destroyed (Kl/starch test paper). The pH of the reaction mixture was 1-2 and was neutralized with 2N sodium hydroxide and extracted with ethyl acetate (4 times with 30 mL). The organic layer was washed with brine, dried over NaSO₄, and concentrated *in vacuo.* The residue obtained was the pure alcohol cis-Bicyclo[4.4.0]decan-1-ol (1.50 gams, 90%). (*M-1: 153.1)*

### Example 4

### trans-4-Methyl-1-(phenylmethyl)-3-Piperidinol

To a solution of alkene 1-Benzyl-4-methyl-1,2,3,6-tetrahydropyridine (18.7 grams, 100 mmol) in tetrahydrofuran (150 mL) was added sodium borohydride (6.5 grams, 170 mmol) at room temperature under nitrogen. The slurry was cooled to 0°C, and BF₃·OEt₂ (15 mL, 16.8 grams, 118.3 mmol) in tetrahydrofuran (25 mL) was slowly added through an addition funnel. The addition was kept slow enough to keep the temperature of the reaction mixture below 0°C. After the addition; the reaction mixture was stirred at 0°C for 1hour and room temperature for 1.5 hours. The reaction was re-cooled to 0°C and water (50 mL) was added slowly to destroy the excess borane. The reaction was stirred at room temperature for 2 hours, followed by the addition of Oxone® (110 grams, 342.8 mmol) in water (500 mL) at 0°C. The reaction mixture was allowed to warm to room temperature and stirred overnight. The reaction was quenched by adding NaHSO₃ (solid) until all excess oxidant was destroyed (Kl/starch test paper). The pH of the reaction mixture was 1-2. The reaction mixture was extracted with ethyl acetate (3 times with 50 mL), and the aqueous layer was adjusted to pH of 12 with 6N sodium hydroxide and extracted with ethyl acetate (4 times with 100 mL). The organic layer was washed with brine, dried over NaSO₄, and concentrated *in vacuo.* The residue obtained is pure alcohol *trans*-4-methyl-1-(phenylmethyl)-3-Piperidinol (19.0 grams, 92%). *(M+1:206.3)*.

### Example 5

### 2-(4-Bromophenyl)ethanol

To a solution of 1-bromo-4-ethenylbenzene (1.0 grams, 5.46 mmol) in tetrahydrofuran (10 mL) was added sodium borohydride (0.35 grams, 9.28 mmol) at room temperature under nitrogen. The slurry was cooled to 0°C, and BF₃·OEt₂ (1.2 mL, 1.32 grams, 9.3 mmol) in tetrahydrofuran (1.50 mL) was slowly added through an addition funnel. The addition was kept slow enough to keep the temperature of the reaction mixture below 0°C. After the addition; the reaction mixture was stirred at 0°C for 1hour and room temperature for 1.5 hours. The reaction was re-cooled to 0°C and water (8.0 mL) was added slowly to destroy the excess borane. The reaction was stirred at room temperature for 2hours, followed by the addition of Oxone® (11.86 grams, 19.3 mmol) in water (20 mL) at 0°C. The reaction mixture was allowed to warm to room temperature and stirred overnight. The reaction was quenched by adding NaHSO₃ (solid) until all excess oxidant was destroyed (Kl/starch test paper). reaction mixture was extracted with ethyl acetate (3 times with 10 mL), and was adjusted to pH 7 with 2N sodium hydroxide and extracted with ethyl acetate (4 times with 20 mL). The organic layer was washed with brine, dried over NaSO₄, and concentrated *in vacuo.* The residue obtained was the purified by chromatography column eluting with ea/hexane(1:5) to give a mixture of alcohol 2-(4-Bromophenyl)ethanol and 1-(4-Bromophenyl)ethanol (4.6:1) (1.50 grams, 90%). *(M+1: 202.1)*

### Example 6

### β,4-Dimethyl-Benzeneethanol

To a solution of 1-methyl-4-(1-methylethenyl)benzene (1.36 grams, 10.3 mmol) in tetrahydrofuran (15 mL) was added sodium borohydride (0.640 grams, 16.9 mmol) at room temperature under nitrogen. The slurry was cooled to 0°C, and BF₃·OEt₂ (1.5 mL, 1.68 grams, 11.8 mmol) in tetrahydrofuran (2.0 mL) was slowly added through an addition funnel. The addition was kept slow enough to keep the temperature of the reaction mixture below 0°C. After the addition; the reaction mixture was stirred at 0°C for 1 hour and room temperature for 1.5 hours. The reaction was re-cooled to 0°C and water (10.0 mL) was added slowly to destroy the excess borane. The reaction was stirred at room temperature for 2 hours, followed by the addition of Oxone® (21.6 grams, 34.8 mmol) in water (40 mL) at 0°C. The reaction mixture was allowed to warm to room temperature and stirred overnight. The reaction was quenched by adding NaHSO₃ (solid) until all excess oxidant was destroyed (potassium iodide/starch test paper). The pH of the reaction mixture was 1-2 and was adjusted to pH of 7 with 2N sodium hydroxide and extracted with ethyl acetate (4 times with 30 mL). The organic layer was washed with brine, dried over NaSO₄, and concentrated *in vacuo.* The residue obtained was the pure alcohol β,4-dimethyl-Benzeneethanol (1.20 grams, 85%). NMR: 7.1(m,4H), 3.7(d,2H), 2.9(m,1), 2.3(s, 3H), 1.5(s,br,1H), 1.3(d, 3H), compared with *J.Org. Chem.* 1982, 47,4692.

## Claims

1. A process for preparing a compound of the formula 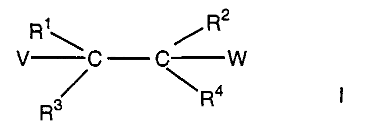 wherein V and W are each independently selected from hydrogen or hydroxy;
R¹, R², R³ and R⁴ are each independently selected from the group consisting of hydrogen, deuterium, amino, halo, hydoxy, nitro, carboxy, trifluoromethyl, trifluoromethoxy, (C₁-C₆)alkyl, (C₁-C₆)alkoxy, (C₃-C₁₀)cycloalkyl wherein the alkyl, alkoxy or cycloalkyl groups are optionally substituted by one to three groups selected from halo, hydroxy, carboxy, amino, (C₁-C₆)alkylamino, ((C₁-C₆)alkyl)₂amino, (C₅-C₉)heteroaryl, (C₂-C₉)heterocycloalkyl, (C₃-C₉)cycloalkyl or (C₆-C₁₀)aryl; or R¹, R², R³ and R⁴ are each independently (C₃-C₁₀)cycloalkyl, (C₃-C₁₀)cycloalkoxy, (C₁-C₆)alkylamino, ((C₁-C₆)alkyl)₂amino, (C₆-C₁₀)arylamino, (C₁-C₆)alkylsulfinyl, (C₆-C₁₀)arylsulfinyl, (C₁-C₆)alkylsulfonyl, (C₆-C₁₀)arylsulfonyl, (C₁-C₆)acyl, (C₁-C₆)alkoxy-CO-NH-, (C₁-C₆)alkyamino-CO-, (C₅-C₉)heteroaryl, (C₂-C₉)heterocycloalkyl or (C₆-C₁₀)aryl wherein the heteroaryl, heterocycloalkyl and aryl groups are optionally substituted by one to three halo, (C₁-C₆)alkyl, (C₁-C₆)alkyl-CO-NH-, (C₁-C₆)alkoxy-CO-NH-, (C₁-C₆)alkyl-CO-NH-(C₁-C₆)alkyl, (C₁-C₆)alkoxy-CO-NH-(C₁-C₆)alkyl, (C₁-C₆)alkoxy-CO-NH-(C₁-C₆)alkoxy, carboxy, carboxy(C₁-C₆)alkyl, carboxy(C₁-C₆)alkoxy, benzyloxycarbonyl(C₁-C₆)alkoxy, (C₁-C₆)alkoxycarbonyl(C₁-C₆)alkoxy, (C₆-C₁₀)aryl, amino, amino(C₁-C₆)alkyl, (C₁-C₆)alkoxycarbonylamino, (C₆-C₁₀)aryl(C₁-C₆)alkoxycarbonylamino, (C₁-C₆)alkylamino, ((C₁-C₆)alkyl)₂amino, (C₁-C₆)alkylamino(C₁-C₆)alkyl, ((C₁-C₆)alkyl)₂amino(C₁-C₆)alkyl, hydroxy, (C₁-C₆)alkoxy, carboxy, carboxy(C₁-C₆)alkyl, (C₁-C₆)alkoxycarbonyl, (C₁-C₆)alkoxycarbonyl(C₁-C₆)alkyl, (C₁-C₆)alkoxy-CO-NH-, (C₁-C₆)alkyl-CO-NH-, cyano, (C₅-C₉)heterocycloalkyl, amino-CO-NH-, (C₁-C₆)alkylamino-CO-NH-, ((C₁-C₆)alkyl)₂amino-CO-NH-, (C₆-C₁₀)arylamino-CO-NH-, (C₅-C₉)heteroarylamino-CO-NH-, (C₁-C₆)alkylamino-CO-NH-(C₁-C₆)alkyl,((C₁-C₆)alkyl)₂amino-CO-NH-(C₁-C₆)alkyl, (C₆-C₁₀)arylamino-CO-NH-(C₁-C₆)alkyl, (C₅-C₉)heteroarylamino-CO-NH-(C₁-C₆)alkyl, (C₁-C₆)alkylsulfonyl, (C₁-C₆)alkylsulfonylamino, (C₁-C₆)alkylsulfonylamino(C₁-C₆)alkyl, (C₆-C₁₀)arylsulfonyl, (C₆-C₁₀)arylsulfonylamino, (C₆-C₁₀)arylsulfonylamino(C₁-C₆)alkyl, (C₁-C₆)alkylsulfonylamino, (C₁-C₆)alkylsulfonylamino(C₁-C₆)alkyl, (C₅-C₉)heteroaryl or (C₂-C₉)heterocycloalkyl;
or R¹ and R², and/or R³ and R⁴, and/or R¹ and R³, and/or R² and R⁴ are taken together with the carbon to which they are attached to form a (C₃-C₁₀)cycloalkyl or (C₂-C₉)heterocycloalkyl group optionally substituted by one to five groups consisting of carboxy, cyano, amino, deuterium, hydroxy, (C₁-C₆)alkyl, (C₁-C₆)alkoxy, halo, (C₁-C₆)acyl, (C₁-C₆)alkylamino, amino(C₁-C₆)alkyl, (C₁-C₆)alkoxy-CO-NH, (C₁-C₆)alkylamino-CO-, (C₂-C₆)alkenyl, (C₂-C₆) alkynyl, (C₁-C₆)alkylamino, amino(C₁-C₆)alkyl, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R¹⁵R¹⁶N-CO-O-, R¹⁵R¹⁶N-CO-(C₁-C₆)alkyl, (C₁-C₆)alkyl-S(O)ₘ, R¹⁵R¹⁶NS(O)ₘ, R¹⁵R¹⁶NS(O)ₘ (C₁-C₆)alkyl, R¹⁵S(O)ₘ R¹⁶N, R¹⁵S(O)ₘR¹⁶N(C₁-C₆)alkyl wherein m is 1 or 2 and R¹⁵ and R¹⁶ are each independently selected from hydrogen or (C₁-C₆)alkyl; or a group of the formula 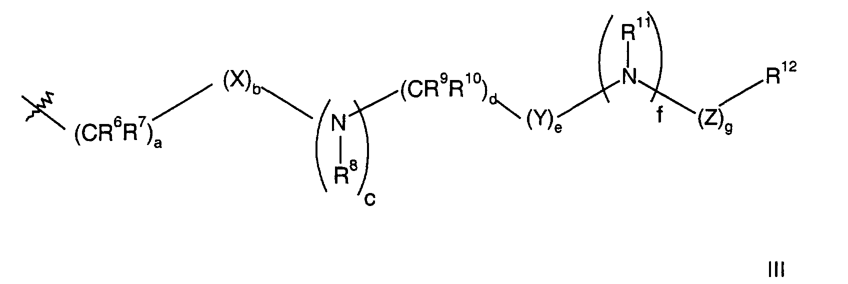 wherein a is 0, 1, 2, 3 or 4;
b, c, e, f and g are each independently 0 or 1;
d is 0, 1, 2, or 3;
X is S(O)ₙ wherein n is 1 or 2; oxygen, carbonyl or -C(=N-cyano)-;
Y is S(O)ₙ wherein n is 1 or 2; or carbonyl; and
Z is carbonyl, C(O)O-, C(O)NR- or S(O)ₙ wherein n is 1 or 2;
R⁶, R⁷, R⁸, R⁹, R¹⁰ and R¹¹ are each independently selected from the group consisting of hydrogen or (C₁-C₆)alkyl optionally substituted by deuterium, hydroxy, amino, trifluoromethyl, (C₁-C₆)acyloxy, (C₁-C₆)acylamino, (C₁-C₆)alkylamino, ((C₁-C₆)alkyl)₂amino, cyano, cyano(C₁-C₆)alkyl, trifluoromethyl(C₁-C₆)alkyl, nitro, nitro(C₁-C₆)alkyl or (C₁-C₆)acylamino;
R¹² is carboxy, cyano, amino, oxo, deuterium, hydroxy, trifluoromethyl, (C₁-C₆)alkyl, trifluoromethyl(C₁-C₆)alkyl, (C₁-C₆)alkoxy, halo, (C₁-C₆)acyl, (C₁-C₆)alkylamino, ((C₁-C₆)alkyl)₂ amino, amino(C₁-C₆)alkyl, (C₁-C₆)alkoxy-CO-NH, (C₁-C₆)alkylamino-CO-, (C₁-C₆)alkylamino, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R¹⁵R¹⁶N-CO-O-, R¹⁵R¹⁶N-CO-(C₁-C₆)alkyl, R¹⁵C(O)NH, R¹⁵OC(O)NH, R¹⁵NHC(O)NH, (C₁-C₆)alkyl-S(O)ₘ, (C₁-C₆)alkyl-S(O)ₘ-(C₁-C₆)alkyl, R¹⁵R¹⁶NS(O)ₘ, R¹⁵R¹⁶NS(O)ₘ (C₁-C₆)alkyl, R¹⁵S(O)ₘ R¹⁶N, R¹⁵S(O)ₘR¹⁶N(C₁-C₆)alkyl wherein m is 1 or 2 and R¹⁵ and R¹⁶ are each independently selected from hydrogen or (C₁-C₆)alkyl; or R¹² is (C₆-C₁₀)aryl, (C₂-C₉)heteroaryl, (C₃-C₁₀)cycloalkyl, (C₂-C₉)heterocycloalkyl, wherein the aryl, heteroaryl, cycloalkyl and heterocycloalkyl groups are optionally substituted by one to four groups consisting of hydrogen, deuterium, halo, (C₁-C₆)alkyl, (C₁-C₆)alkyl-CO-NH-, (C₁-C₆)alkoxy-CO-NH-, (C₁-C₆)alkyl-CO-NH-(C₁-C₆)alkyl, (C₁-C₆)alkoxy-CO-NH-(C₁-C₆)alkyl, (C₁-C₆)alkoxy-CO-NH-(C₁-C₆)alkoxy, carboxy, carboxy(C₁-C₆)alkyl, carboxy(C₁-C₆)alkoxy, benzyloxycarbonyl(C₁-C₆)alkoxy, (C₁-C₆)alkoxycarbonyl(C₁-C₆)alkoxy, (C₆-C₁₀)aryl, amino, amino(C₁-C₆)alkyl, (C₁-C₆)alkoxycarbonylamino, (C₆-C₁₀)aryl(C₁-C₆)alkoxycarbonylamino, (C₁-C₆)alkylamino, ((C₁-C₆)alkyl)₂amino, (C₁-C₆)alkylamino(C₁-C₆)alkyl, ((C₁-C₆)alkyl)₂amino(C₁-C₆)alkyl, hydroxy, (C₁-C₆)alkoxy, carboxy, carboxy(C₁-C₆)alkyl, (C₁-C₆)alkoxycarbonyl, (C₁-C₆)alkoxycarbonyl(C₁-C₆)alkyl, (C₁-C₆)alkoxy-CO-NH-, (C₁-C₆)alkyl-CO-NH-, cyano, (C₅-C₉)heterocycloalkyl, amino-CO-NH-, (C₁-C₆)alkylamino-CO-NH-, ((C₁-C₆)alkyl)₂amino-CO-NH-, (C₆-C₁₀)arylamino-CO-NH-, (C₅-C₉)heteroarylamino-CO-NH-, (C₁-C₆)alkylamino-CO-NH-(C₁-C₆)alkyl, ((C₁-C₆)alkyl)₂amino-CO-NH-(C₁-C₆)alkyl, (C₆-C₁₀)arylamino-CO-NH-(C₁-C₆)alkyl, (C₅-C₉)heteroarylamino-CO-NH-(C₁-C₆)alkyl, (C₁-C₆)alkylsulfonyl, (C₁-C₆)alkylsulfonylamino, (C₁-C₆)alkylsulfonylamino(C₁-C₆)alkyl, (C₆-C₁₀)arylsulfonyl, (C₆-C₁₀)arylsulfonylamino, (C₆-C₁₀)arylsulfonylamino(C₁-C₆)alkyl, (C₁-C₆)alkylsulfonylamino, (C₁-C₆)alkylsulfonylamino(C₁-C₆)alkyl, (C₅-C₉)heteroaryl or (C₂-C₉)heterocycloalkyl;
with the proviso that X and Y cannot both be hydrogen or hydroxy;
comprising reacting a compound of the formula 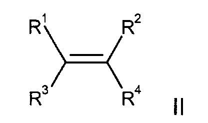 wherein R¹, R², R³ and R⁴ are as defined as above, with a borane, followed by reacting the intermediate so formed with Oxone® .

2. A process according to claim 1, wherein the borane is borane, diborane, or (C₁-C₁₂)alkylborane.

3. A process according to claim 1 or claim 2, wherein R¹, R², R³ and R⁴ are each independently selected from hydrogen, deuterium, (C₃-C₁₀)cycloalkyl, (C₃-C₁₀)cycloalkoxy, (C₁-C₆)alkylsulfonyl, (C₆-C₁₀)arylsulfonyl, (C₁-C₆)acyl, (C₃-C₁₀)cycloalkyl, (C₅-C₉)heteroaryl, (C₂-C₉)heterocycloalkyl, (C₆-C₁₀)aryl, wherein heteroaryl, heterocycloalkyl, aryl groups are optionally substituted by one to three halo, (C₁-C₆)alkyl or (C₁-C₆)alkoxy.

4. A process according to claim 1 or claim 2, wherein R¹ and R², or R³ and R⁴, R¹ and R³, or R² and R⁴ are taken together with the carbon to which they are attached to form a (C₃-C₁₀)cycloalkyl or (C₂-C₉)heterocycloalkyl group optionally substituted by one of five groups consisting of carboxy, cyano, amino, deuterium, hydroxy, (C₁-C₆)alkyl, (C₁-C₆)alkoxy, halo, (C₁-C₆)acyl, (C₁-C₆)alkylamino, amino(C₁-C₆)alkyl, (C₁-C₆)alkoxy-CO-NH, (C₁-C₆)alkylamino-CO-, (C₂-C₆)alkenyl, (C₂-C₆)alkynyl, (C₁-C₆)alkylamino, amino(C₁-C₆)alkyl, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R¹⁵R¹⁶N-CO-O-, R¹⁵R¹⁶N-CO-(C₁-C₆)alkyl, (C₁-C₆)alkyl-S(O)ₘ, R¹⁵R¹⁶NS(O)ₘ, R¹⁵R¹⁶NS(O)ₘ(C₁-C₆)alkyl, R¹⁵S(O)ₘR¹⁶N, R¹⁵S(O)ₘR¹⁶N(C₁-C₆)alkyl wherein m is 1 or 2 and R¹⁵ and R¹⁶ are each independently selected from hydrogen or (C₁-C₆)alkyl; and a group of the formula 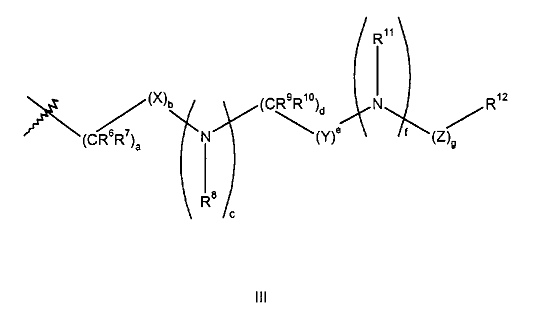 wherein a is 0, 1, 2, 3 or 4;
b, c, e, f and g are each independently 0 or 1;
d is 0, 1, 2, or 3;
X is S(O)ₙ wherein n is 1 or 2; oxygen, carbonyl or -C(=N-cyano)-;
Y is S(O)ₙ wherein n is 1 or 2; or carbonyl; and
Z is carbonyl, C(O)O-, C(O)NR- or S(O)ₙ wherein n is 1 or 2;
R⁶, R⁷, R⁸, R⁹, R¹⁰ and R¹¹ are each independently selected from the group consisting of hydrogen or (C₁-C₆)alkyl optionally substituted by deuterium, hydroxy, amino, trifluoromethyl, (C₁-C₆)acyloxy, (C₁-C₆)acylamino, (C₁-C₆)alkylamino, ((C₁-C₆)alkyl)₂amino, cyano, cyano(C₁-C₆)alkyl, trifluoromethyl(C₁-C₆)alkyl, nitro, nitro(C₁-C₆)alkyl or (C₁-C₆)acylamino.

5. A process according to any one of claims 1 to 4, wherein a is 0; b is 1; X is carbonyl; c is 0; d is 0; e is 0; f is 0; and g is 0.

6. A process according to any one of claims 1 to 4, wherein a is 0; b is 1; X is carbonyl; c is 0; d is 1; e is 0; f is 0; and g is 0.

7. A process according to any one of claims 1 to 4, wherein a is 0; b is 1; X is carbonyl; c is 1; d is 0; e is 0; f is 0; and g is 0.

8. A process according to any one of claims 1 to 4, wherein a is 0; b is 1; X is ― C(=N=cyano)-; c is 1; d is 0; e is 0; f is 0; and g is 0.

9. A process according to any one of claims 1 to 4, wherein a is 0; b is 0; c is 0; d is 0; e is 0; f is 0; and g is 1; and Z is -C(O)-O-.

10. A process according to any one of claims 1 to 4, wherein a is 0; b is 1; X is S(O)ₙ; n is 2; c is 0; d is 0; e is 0; f is 0; and g is 0.

11. A process according to any one of claims 1 to 4, wherein a is 0; b is 1; X is S(O)ₙ; n is 2; c is 0; d is 2; e is 0; f is 1; g is 1; and Z is carbonyl.

12. A process according to any one of claims 1 to 4, wherein a is 0; b is 1; X is S(O)ₙ; n is 2; c is 0; d is 2; e is 0; f is 1; and g is 0.

13. A process according to any one of claims 1 to 4, wherein a is 0; b is 1; X is carbonyl; c is 1; d is 0; e is 1; Y is S(O)ₙ ; n is 2; f is 0; and g is 0.

14. A process according to any one of claims 1 to 4, wherein a is 0; b is 1; X is S(O)ₙ; n is 2; c is 1; d is 0; e is 0; f is 0; and g is 0.

15. A process according to any one of claims 1 to 4, wherein a is 1; b is 1; X is carbonyl; c is 1; d is 0; e is 0; f is 0; and g is 0.

16. A process according to any one of claims 1 to 4, wherein a is 0; b is 1; X is S(O)ₙ; c is 0; d is 1; e is 1; Y is S(O)ₙ; n is 2; f is 0; and g is 0.

17. A process according to any one of claims 1 to 4, wherein a is 0; b is 1; X is S(O)ₙ; c is 0; d is 2, 3 or 4; e is 1; Y is S(O)ₙ; n is 2; f is 1; and g is 0.

18. A process according to any one of claims 1 to 4, wherein a is 0; b is 1; X is oxygen; c is 0; d is 2,3 or 4; e is 1; Y is S(O)ₙ; n is 2; f is 1; and g is 0.

19. A process according to any one of claims 1 to 4, wherein a is 0; b is 1; X is oxygen; c is 0; d is 2, 3 or 4; e is 1; Y is S(O)ₙ; n is 2; f is 0; and g is 0.

20. A process according to any one of claims 1 to 4, wherein a is 0; b is 1; X is carbonyl; c is 1; d is 2, 3 or 4; e is 1; Y is S(O)ₙ; f is 0; and g is 0.

21. A process according to any one of claims 1 to 4, wherein a is 0; b is 1; X is carbonyl; c is 1; d is 2, 3 or 4; e is 1; Y is S(O)ₙ; n is 2; f is 1; and g is 0.

22. A process according to any of claims 1 to 22, wherein R¹² is cyano, deuterium, hydroxy, trifluoromethyl, (C₁-C₆)alkyl, trifluoromethyl(C₁-C₆)alkyl, (C₁-C₆)alkoxy, halo, (C₁-C₆)acyl, (C₆-C₁₀)aryl, (C₂-C₉)heteroaryl, (C₃-C₁₀)cycloalkyl, or (C₂-C₉)heterocycloalkyl.
